# FASCICULE DE BREVET EUROPEEN

(11) **EP 2 029 095 B1**
(45) Date de publication et mention de la délivrance du brevet: **30.08.2017**
(21) Numéro de dépôt: 07765946.4
(22) Date de dépôt: 23.05.2007
(51) Int. Cl.: A61K 8/42, A61K 8/365, A61K 8/43, A61Q 5/04

(54) **PROCEDE DE DEFRISAGE DES FIBRES KERATINIQUES AVEC UN MOYEN DE CHAUFFAGE ET DES AGENTS DENATURANTS**
VERFAHREN ZUR GLÄTTUNG VON KERATINFASERN MIT ERWÄRMUNGSMITTEL UND DENATURIERUNGSSTOFFEN
PROCESS FOR STRAIGHTENING KERATIN FIBRES WITH A HEATING MEANS AND DENATURING AGENTS

(30) Priorité: 24.05.2006 FR 0651911; 19.06.2006 US 814529 P
(43) Date de publication de la demande: 04.03.2009
(73) Titulaire: L'Oréal, 75008 Paris (FR)
(72) Inventeur: PHILIPPE, Michel, F-91320 Wissous (FR); MALLE, Gérard, F-77580 Villiers S/Morin (FR); BARBARAT, Philippe, F-92270 BOIS-COLOMBES (FR)
(74) Mandataire: Rivière, François Armand
(86) Numéro de dépôt international: PCT/FR2007/000870
(87) Numéro de publication internationale: WO 2007/135297

(56) Documents cités:
- EP-A- 1 532 963
- EP-A- 1 535 598
- EP-A- 1 584 327
- US-A- 4 314 572
- US-A- 5 957 140
- US-A1- 2003 143 173
- DATABASE WPI Week 200414 Derwent Publications Ltd., London, GB; AN 2004-137526 XP002417880 & JP 2004 002459 A (MIRUBON KK) 8 janvier 2004 (2004-01-08)
- DATABASE WPI Week 200346 Derwent Publications Ltd., London, GB; AN 2003-485967 XP002417881 & JP 2002 356408 A (MIRUBON KK) 13 décembre 2002 (2002-12-13)

## Description

L'invention a pour objet un procédé de défrisage des fibres kératiniques avec un moyen de chauffage et au moins deux agents dénaturants.

Le procédé de défrisage selon l'invention est réalisé sans utiliser d'agent réducteur, ni d'agent de lanthionisation. Il ne comprend aucune étape de réduction, ni de lanthionisation.

Par *"fibres kératiniques,",* on entend, selon l'invention, des fibres d'origine humaine ou animale telles que les cheveux, les poils, les cils, la laine, l'angora, le cachemire ou la fourrure. Bien que l'invention ne soit pas limitée à des fibres kératiniques particulières, il sera néanmoins fait plus particulièrement référence aux cheveux.

Le terme *"défrisage"* englobe, selon l'invention, le défrisage, le lissage ou le décrêpage de cheveux caucasiens ou africains. Le terme « *défriser* » englobe, selon l'invention, défriser, lisser ou décrêper des cheveux caucasiens ou africains.

On entend par « *agent dénaturant »,* un composé organique ou minéral possédant à la fois au moins un site donneur d'électrons à caractère basique ou nucléophile et au moins un site accepteur d'électrons à caractère acide ou électrophile, interagissant avec les liaisons faibles de la kératine.

Selon l'invention, un agent dénaturant est un composé capable de diminuer le pouvoir rotatoire d'une protéine modèle telle que par exemple la bovine serum albumine d'au moins 7° et/ou 5° à 579nm, les mesures étant effectuées après 3h d'incubation à 37°C, à l'aide d'un polarimètre, tel que décrit dans Biochemistry 2 (1), 47-57, 1963 :
- soit dans un tampon TRIS 0.05M pH 7,6
- soit dans une solution d'urée 5,45M lorsque la solubilité du composé est insuffisante dans le tampon TRIS 0.05M pH 7,6.

Le composé est considéré comme agent dénaturant selon l'invention si la diminution du pouvoir rotatoire est d'au moins 7° dans le tampon TRIS 0.05M pH 7,6 et/ou d'au moins 5° dans la solution d'urée 5,45M.

Par « *liaisons faibles de la kératine »,* on entend l'ensemble des liaisons non covalentes telles que :
- les liaisons salines résultant d'interactions coulombiennes entre les groupes fonctionnels présents au niveau des chaînes latérales des acides aminés
- les liaisons hydrogènes qui s'établissent entre les acides aminés par l'intermédiaire notamment des atomes d'oxygène et d'hydrogène
- les liaisons hydrophobes qui résultent de la tendance des chaînes non polaires des acides aminés à s'associer pour minimiser les contacts avec l'eau

Par « *moyen de chauffage»,* on entend tout moyen permettant de chauffer les fibres kératiniques à une température d'au moins 110°C tels que les fers chauffants, par exemple les fers plats ou ronds, les générateurs de micro-ondes, les sources de rayonnement infra-rouge.

Deux techniques sont utilisées pour obtenir une déformation permanente des cheveux. Elles sont basées sur une rupture des liaisons covalentes disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée. Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ▪ La première réaction consiste en une bêta-élimination sur la cystine provoquée par un ion hydroxyde, conduisant à la rupture de cette liaison et à la formation de déhydro-alanine. 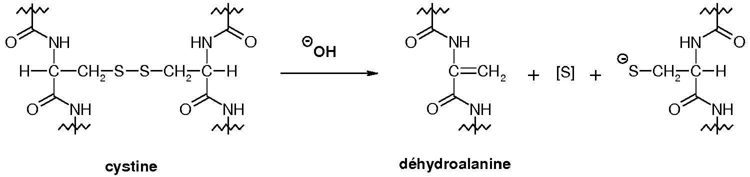
   ▪ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

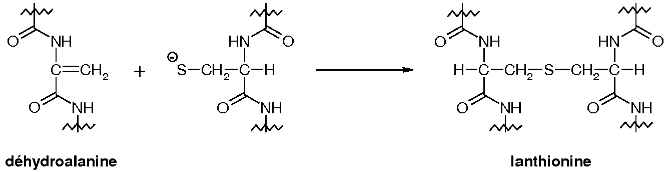

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente ("hair waving"). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.

Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais "lye relaxers" et celles n'en contenant pas sont dénommées " no-lye relaxers ".

Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions nettement moins agressives pour le cheveu.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le lissage.

Il a également été proposé dans de très nombreux brevets d'associer des agents réducteurs habituels (thiols ou sulfites ou bisulfites) avec de l'urée ou des alkylurées pour diminuer l'irritation et les dommages causés aux cheveux aussi bien pour la mise en forme que pour le défrisage. On citera par exemple :
- la demande CA 1315204 qui décrit une composition contenant du thioglycolate d'ammonium (5,5-11,5%) et de l'urée ou une monoalkylurée (1-3%) pour la mise en forme des cheveux,
- la demande US 3847165 qui décrit une composition contenant du thioglycolate d'ammonium (1,2-1,4M) et de l'urée (2,0-2,7M) pour la mise en forme des cheveux à un pH acide,
- la demande NL 6410355 qui décrit une composition contenant un sulfite (0,8-1,5M) et de l'urée (0,6-3,0M) pour la mise en forme et le défrisage des cheveux,
- la demande JP 2000/229819 qui décrit une composition contenant un sulfite ou bisulfite (0,5-15%), de l'urée (0,5-15%) et un alcool (éthanol et/ou isopropanol, 1-30%) pour la mise en forme et le défrisage des cheveux,

Il a également été proposé dans de très nombreux brevets d'associer des hydroxydes, servant d'actif de lanthionisation, avec certains additifs servant généralement à protéger les cheveux. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages.

Si toutes ces propositions conduisent à des améliorations plus ou moins marquées, elles ne permettent pas de diminuer de façon suffisante les dégâts liés à la causticité même des hydroxydes.
En outre,
- les documents EP 1 584 327, JP-A-2004002459. JP-A-2002356408 décrivent un procédé de défrisage des cheveux impliquant l'application d'une composition de défrisage et la mise en oeuvre d'un chauffage_{.}
- le document US-A-2003/0143173 divulgue que les acides alcanoïques sont capables de défriser les cheveux,
- les documents EP-A-1535 598 et EP-A-1 532 963, déposés par le Demanderesse, divulguant des procédés de défrisage impliquant l'application d'une guanidine ainsi qu'une étape de chauffage, et
- le document US-A-4 314 572 divulgue le défrisage au moyen de guanidine, sans chauffage.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

L'utilisation du résorcinol à la concentration de 40% et à un pH de 7 pour défriser le cheveu a été rapportée par M. WONG et al. : M.WONG, G.VIS-SUREL,and J.EPPS J. Soc. Cosmet. Chem. (1994), 45 , 347-352. Toutefois, les essais que nous avons réalisés dans ces conditions sur des cheveux africains naturellement frisés ne les défrisent pas mais conduisent, tout au plus, à une légère détente.

Après d'importantes études, on vient maintenant de découvrir, de façon tout à fait surprenante et inattendue, que l'on pouvait défriser durablement le cheveu en associant l'action d'au moins deux agents dénaturants et d'un moyen de chauffage à une température supérieure à 110°C. On obtient ainsi d'excellents résultats en terme de défrisage, de propriétés cosmétiques des cheveux et d'intégrité de la fibre.

Sans être liée par la théorie, la Demanderesse pense qu'il existe une action conjointe, sur les fibres kératiniques, d'au moins deux agents dénaturants et d'un moyen de chauffage, qui permet de les défriser de façon efficace et durable.

La Demanderesse a trouvé qu'il était possible de remédier aux inconvénients de l'art antérieur et de remplir les objectifs précités en mettant en oeuvre un procédé de défrisage des fibres kératiniques comprenant :
- une étape d'application sur les fibres kératiniques d'une composition de défrisage contenant au moins deux agents dénaturants au moins un agent dénaturant étant une guanidine de formule (III) telle que définie ci-après et au moins un agent dénaturant dérivé d'α-acétoacide de formule (V) telle que définie ci-après,
- une étape d'élévation de la température des fibres kératiniques, à l'aide d'un moyen de chauffage, à une température comprise entre 110 et 250°C.

Avantageusement, les agents dénaturants sont de masse molaire supérieure à 18,1 g/mol, de préférence entre 40 et 600 g/mol.

De préférence, la composition de défrisage comprend une concentration globale en agents dénaturants comprise entre 1M et 8M, plus avantageusement entre 2M et 8M desdits agents dénaturants ; ce qui correspond à une concentration pondérale comprise entre environ 6% et environ 80%, plus avantageusement entre environ 12% et environ 80%, par rapport au poids total de la composition, desdits agents dénaturants.

Avantageusement, on élève la température à l'aide du moyen de chauffage à une température comprise entre 120°C et 220°C, plus avantageusement entre 140°C et 220°C.

Selon un mode de réalisation, ladite composition est appliquée sur des fibres kératiniques humides.

On peut également avantageusement intercaler entre l'étape d'application de la composition et l'étape d'élévation de température, une étape destinée à éliminer l'excédant de la composition, par exemple au moyen d'une serviette.

Par "guanidine", utilisable à titre d'actif de défrisage, on entend tout dérivé comprenant dans sa formule chimique au moins un atome de carbone doublement lié à un atome d'azote et simplement lié à deux autres atomes d'azote. Ces guanidines sont sélectionnées parmi les composés de formule générale (III) ci-dessous : 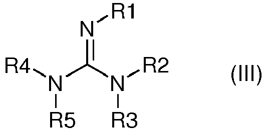 dans laquelle :
R1, R2, R3, R4, R5 représentent, indépendamment, :
   (i) un atome d'hydrogène ou
   (ii) un radical alkyle ou alcényl inférieur en C1-C4, linéaire ou ramifié, éventuellement substitué par un ou 2 radicaux choisi parmi : hydroxyle, amino, diméthylamino, méthoxy, éthoxy, carboxyle, carboxamide, N-méthylcarboxamide ou SO3H,
et leurs sels organiques ou minéraux

Parmi les composés de formule (III), on peut notamment citer les composés préférés suivants :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- la 1-méthylguanidine, chlorhydrate
- la 1,1-diméthylguanidine, chlorhydrate
- la 1-éthylguanidine, chlorhydrate
- la 1,1-diéthylguanidine, chlorhydrate
- la 1,1,3,3-tétraméthylguanidine, chlorhydrate
- la 2-tert-butyl-1,1,3,3- tétraméthylguanidine, chlorhydrate

Parmi les composés de formule (III), on peut notamment citer les composés particulièrement préférés suivants :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- la 1-méthylguanidine, chlorhydrate
- la 1,1-diméthylguanidine, chlorhydrate
- la 1-éthylguanidine, chlorhydrate
- la 1,1,3,3-tétraméthylguanidine, chlorhydrate
- la 2-tert-butyl-1,1,3,3- tétraméthylguanidine, chlorhydrate

Par dérivés d'α-hydroxyacides utilisable à titre d'actif de défrisage, on entend tout dérivé sélectionné parmi les composés de formules générales (V) ci-dessous :

Définition des dérivés d'α-cétoacides de formule générale (V) :

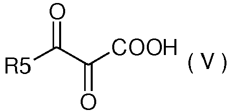

R5 représente COOH, un radical alkyle linéaire ou ramifié en C1-C6 éventuellement substitué par un radical OH ou COOH ou Br ; un radical phényle ou benzyle éventuellement substitués par un radical OH ou COOH ; ou encore un radical indolyl ou 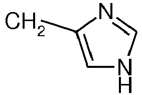 et leurs stéréoisomères et sels organiques ou minéraux et solvates.

Les composés de formule (V) préférés sont choisis parmi:
L' acide pyruvique
L'acide 2-cétobutyrique
L'acide ß-hydroxypyruvique
L'acide 3-méthyl-2-oxobutyrique
L'acide 2-oxovalérique
L'acide cétomalonique
L'acide 3-méthyl-2oxovalérique
L'acide triméthylpyruvique
L'acide oxolacétique
L'acide 2- cétoglutarique
L'acide benzylformique
L'acide 2-oxooctanoïque
L'acide 2-oxoadipique
L'acide phénylpyruvique
L'acide bromopyruvique
L'acide 2- cétopimélique
L'acide 4-hydroxyphénylpyruvique
L'acide 3-indoleglyoxalique
L'acide imidazolepyruvique, HCl
L'acide 2-céto L- gulonique
L'acide 2-carboxy-α-oxobenzène acétique
L'acide indole-3 pyruvique
L'acide 2-cétoglutarique dihydrate
Le pyruvamide
Le N-méthyl- pyruvamide
Le N-éthyl- pyruvamide
Le N,N-diméthyl-pyruvamide
Le N-2-hydoxyéthyl- pyruvamide
et leurs stéréoisomères et sels organiques ou minéraux et solvates.

Les composés de formule (V) particulièrement préférés sont choisi parmi:
L' acide pyruvique
L'acide 2-cétobutyrique
L'acide ß-hydroxypyruvique
L'acide cétomalonique
L'acide oxolacétique
L'acide 2- cétoglutarique
L'acide 2-céto L- gulonique
L'acide 2-cétoglutarique dihydrate
Le pyruvamide
et leurs stéréoisomères et sels organiques ou minéraux et solvates

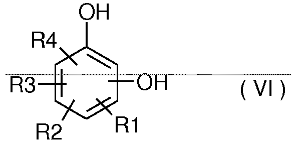

Dans les compositions selon l'invention destinées à un processus de défrisage, décrêpage ou lissage des cheveux, le mélange en toutes proportions d'au moins deux agents dénaturants tels que définis précédemment est avantageusement présent à une concentration molaire globale comprise entre 1M et 8M, plus avantageusement, à une concentration comprise entre 2M et 8M.

Dans le procédé selon l'invention et dans le kit, le pH des compositions est inférieur à 9, et plus préférentiellement inférieur à 7.

Les compositions selon l'invention se présentent soit sous la forme d'une solution aqueuse, soit sous forme d'une crème épaissie de façon à maintenir les cheveux aussi raides que possible. On réalise ces crèmes, sous forme d'émulsions « lourdes ».

Dans le but d'améliorer les propriétés cosmétiques des fibres kératiniques ou encore d'atténuer ou d'éviter leur dégradation, la composition utilisée selon l'invention peut également comprendre un ou plusieurs actifs cosmétiques supplémentaires. Généralement, le ou lesdits actifs cosmétiques supplémentaires représentent de 0,01 à 30%, de préférence de 0,1 à 10%, en poids du poids total de la composition cosmétique.

Généralement, la composition appliquée sur les fibres kératiniques est appliquée à hauteur de 0,05 à 20 g, de préférence de 0,1 à 10 g de composition par gramme de fibre kératinique sèche.

Après application de la composition, et avant l'élévation de la température des fibres kératiniques au moyen d'un moyen de chauffage, on peut laisser poser ladite composition, généralement pendant 30 secondes à 60 minutes, de préférence 5 à 45 minutes.

Le procédé selon l'invention comprend, après l'étape d'application de la composition, une étape d'élévation de la température des fibres kératiniques, au moyen d'un moyen de chauffage, à une température comprise entre 110°C et 250°C.

Avantageusement, on utilise un fer comme moyen de chauffage.

Au sens de la présente invention, on entend par «fer» un dispositif de chauffage des fibres kératiniques mettant en contact lesdites fibres et le dispositif de chauffage, l'extrémité du fer venant en contact avec les cheveux présente généralement deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut être effectuée par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres kératiniques avant l'étape d'élévation de la température, de manière à éviter d'importants dégagements de vapeurs qui pourraient brûler les mains du coiffeur et le cuir chevelu de la personne. Cette étape de pré-séchage peut se faire par exemple au moyen d'un séchoir, d'un casque, ou bien encore par séchage libre.

L'invention concerne aussi un kit comprenant au moins :
- un moyen de chauffage procurant une température comprise entre 110 et 250°C.
- une composition de défrisage contenant au moins deux agents dénaturants tels que définis précédemment.

L'invention pourra être mieux comprise à l'aide de l'exemple non limitatif qui sui et qui constitue un mode de réalisation préférentiel des compositions selon l'invention.

Les compositions pouvant être appliquées en mélange (voir exemple 1) ou de façon successive

### EXEMPLE 1:

On réalise une composition de défrisage simplifiée contenant un mélange de chlorhydrate de guanidine à une concentration de 2M et d'acide pyruvique à une concentration de 2M , dans l'eau, en tant qu'actif de défrisage. On applique cette composition sur des cheveux africains naturellement frisés pendant 15 minutes à une température de 40°C puis on essuie rapidement les cheveux avec une serviette.

On procède ensuite à un lissage mèche à mèche de la chevelure à l'aide d'un fer plat chauffé à 180°C pendant 10 à 15 secondes. Les cheveux sont défrisés efficacement et doux au toucher.

## Revendications

1. Procédé de défrisage des fibres kératiniques comprenant :
(i) une étape d'application sur les fibres kératiniques d'une composition de défrisage ayant un pH inférieur à 9 contenant au moins deux agents dénaturants ;
- au moins un des agents dénaturants est une guanidine répondant à la formule générale (III) ci-dessous : 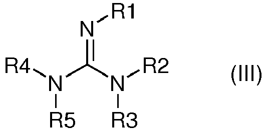 dans laquelle :
R1, R2, R3, R4, R5 représentent, indépendamment :
(i) un atome d'hydrogène ou
(ii) un radical alkyle ou alcényl inférieur en C1-C4, linéaire ou ramifié, éventuellement substitué par un ou 2 radicaux choisi parmi : hydroxyle, amino, diméthylamino, méthoxy, éthoxy, carboxyle, carboxamide, N-méthylcarboxamide ou SO3H,
et leurs sels organiques ou minéraux ;
- et au moins un agent dénaturant choisi parmi les dérivés d'α-cétoacides répondant à la formule générale (V) : 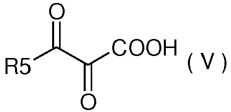
- R5 représente COOH, alkyle linéaire ou ramifié en C1-C6 éventuellement substitué par un radical OH ou COOH ou Br ; phényle ou benzyle éventuellement substitués par un radical OH ou COOH ; ou encore un radical indolyl ou 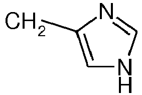
- et leurs stéréoisomères et sels organiques ou minéraux et solvates ;
(ii) une étape d'élévation de la température des fibres kératiniques, à l'aide d'un moyen de chauffage, à une température comprise entre 110 et 250°C.

2. Procédé selon la revendication 1, **caractérisé par le fait que** le pH de la composition est inférieur à 7.

3. Procédé selon la revendication 1 ou 2, **caractérisé par** le fait la composition de défrisage comprend une concentration globale comprise entre 1M et 8M, de préférence entre 2M et 8M desdits agents dénaturants.

4. Procédé selon l'une quelconque des revendications 1 à 3, **caractérisé par le fait qu'**on on élève la température à l'aide du moyen de chauffage à une température comprise entre 120°C et 220°C, plus avantageusement entre 140°C et 220°C.

5. Procédé selon l'une quelconque des revendications précédentes, **caractérisé par le fait que**, la masse molaire des dénaturants est comprise entre 40 et 600 g/mol.

6. Procédé selon l'une quelconque des revendications précédentes, **caractérisé par le fait que** la composition est appliquée sur des fibres kératiniques humides.

7. Procédé selon l'une quelconque des revendications précédentes, **caractérisé par le fait que** les composés de formule (III) sont choisis parmi :
- la guanidine, chlorhydrate
- la guanidine, acétate
- la guanidine, sulfate
- la guanidine, carbonate
- la guanidine, bicarbonate
- la guanidine, phosphate
- la guanidine, sulfamate
- la 1-méthylguanidine, chlorhydrate
- la 1,1-diméthylguanidine, chlorhydrate
- la 1-éthylguanidine, chlorhydrate
- la 1,1,3,3-tétraméthylguanidine, chlorhydrate
- la 2-tert-butyl-1,1,3,3- tétraméthylguanidine, chlorhydrate

8. Procédé selon l'une quelconque des revendications précédentes, **caractérisé par le fait que** les composés de formule (V) sont choisis parmi:
L'acide pyruvique
L'acide 2-cétobutyrique
L'acide ß-hydroxypyruvique
L'acide 3-méthyl-2-oxobutyrique
L'acide 2-oxovalérique
L'acide cétomalonique
L'acide 3-méthyl-2oxovalérique
L'acide triméthylpyruvique
L'acide oxolacétique
L'acide 2- cétoglutarique
L'acide benzylformique
L'acide 2-oxooctanoïque
L'acide 2-oxoadipique
L'acide phénylpyruvique
L'acide bromopyruvique
L'acide 2- cétopimélique
L'acide 4-hydroxyphénylpyruvique
L'acide 3-indoleglyoxalique
L'acide imidazolepyruvique, HCl
L'acide 2-céto L- gulonique
L'acide 2-carboxy-α-oxobenzène acétique L'acide indole-3 pyruvique
L'acide 2-cétoglutarique dihydrate
Le pyruvamide
Le N-méthyl-pyruvamide
Le N-éthyl-pyruvamide
Le N,N-diméthyl-pyruvamide
Le N-2-hydoxyéthyl-pyruvamide
et leurs stéréoisomères et sels organiques ou minéraux et solvates.

9. Procédé selon l'une quelconque des revendications précédentes, **caractérisé par le fait que** les composés de formule (V) sont choisis parmi:
L' acide pyruvique
L'acide 2-cétobutyrique
L'acide ß-hydroxypyruvique
L'acide cétomalonique
L'acide oxolacétique
L'acide 2- cétoglutarique
L'acide 2-céto L- gulonique
L'acide 2-cétoglutarique dihydrate
Le pyruvamide
et leurs stéréoisomères et sels organiques ou minéraux et solvates

10. Procédé selon l'une quelconque des revendications précédentes, **caractérisé par le fait que** les fibres kératiniques sont des cheveux.

11. Kit comprenant au moins :
- un moyen de chauffage procurant une température comprise entre 110 et 250°C.
- une composition de défrisage contenant au moins deux agents dénaturants tels que définis selon l'une quelconque des revendications 1 et 7 à 9.

## Patentansprüche

1. Verfahren zur Entkräuselung von Keratinfasern, umfassend:
(i) einen Schritt des Aufbringens einer Entkräuselungszusammensetzung mit einem pH-Wert unter 9, die mindestens zwei Denatürierungsmittel enthält, auf die Keratinfasern;
- wobei es sich bei mindestens einem der Denaturierungsmittel um ein Guanidin der nachstehenden allgemeinen Formel (III): 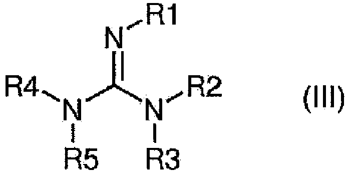 in der:
R1, R2, R3, R4, R5 unabhängig für:
(i) ein Wasserstoffatom oder
(ii) einen geraden oder verzweigten C1-C4-Niederalkyl- oder -alkenylrest, der gegebenenfalls durch einen oder 2 Reste, die aus Hydroxyl, Amino, Dimethylamino, Methoxy, Ethoxy, Carboxyl, Carboxamid, N-Methylcarboxamid oder SO3H ausgewählt sind, substituiert ist stehen,
und deren organische oder anorganische Salze handelt;
- und mindestens ein Denaturierungsmittel, das aus α-Ketosäuren, die der allgemeinen Formel (V) entsprechen: 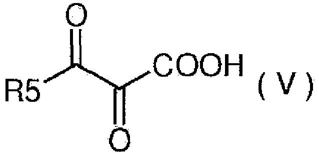 R5 für COOH, lineares oder verzweigtes C1-C6-alkyl, das gegebenenfalls durch einen OH- oder COOH- oder Br-Rest substituiert ist; Phenyl oder Benzyl, das gegebenenfalls durch einen OH- oder COOH-Rest substituiert ist; oder auch einen Indolylrest oder 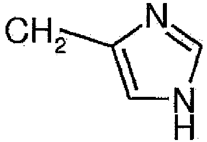 steht,
und deren Stereoisomeren und organischen oder anorganischen Salzen und Solvaten ausgewählt ist;
(ii) einen Schritt des Erhöhens der Temperatur der Keratinfasern mit Hilfe eines Erhitzungsmittels auf eine Temperatur zwischen 110 und 250°C.

2. Verfahren nach Anspruch 1, **dadurch gekennzeichnet, dass** der pH-Wert der Zusammensetzung unter 7 liegt.

3. Verfahren nach Anspruch 1 oder 2, **dadurch gekennzeichnet, dass** die Entkräuselungszusammensetzung eine Gesamtkonzentration der Denaturierungsmittel zwischen 1 M und 8 M, vorzugsweise zwischen 2 M und 8 M, aufweist.

4. Verfahren nach einem der Ansprüche 1 bis 3, **dadurch gekennzeichnet, dass** man die Temperatur mit Hilfe des Erhitzungsmittels auf eine Temperatur zwischen 120°C und 220°C und hoch vorteilhafter zwischen 140°C und 220°C erhöht.

5. Verfahren nach einem der vorhergehenden Ansprüche, **dadurch gekennzeichnet, dass** die Molmasse der Denaturierungsmittel zwischen 40 und 600 g/mol liegt.

6. Verfahren nach einem der vorhergehenden Ansprüche, **dadurch gekennzeichnet, dass** die Zusammensetzung auf feuchte Keratinfasern aufgebracht wird.

7. Verfahren nach einem der vorhergehenden Ansprüche, **dadurch gekennzeichnet, dass** die Verbindungen der Formel III aus:
- Guanidinhydrochlorid,
- Guanidinacetat,
- Guanidinsulfat,
- Guanidincarbonat,
- Guanidinhydrogencarbonat,
- Guanidinphosphat,
- Guanidinsulfamat,
- 1-Methylguanidinhydrochlorid,
- 1,1-Dimethylguanidinhydrochlorid,
- 1-Ethylguanidinhydrochlorid,
- 1,1,3,3-Tetramethylguanidinhydrochlorid und
- 2-tert-Butyl-1,1,3,3-tetramethylguanidinhydrochlorid
ausgewählt sind.

8. Verfahren nach einem der vorhergehenden Ansprüche, **dadurch gekennzeichnet, dass** die Verbindungen der Formel (V) aus:
Brenztraubensäure,
2-Ketobuttersäure,
β-Hydroxybrenztraubensäure,
3-Methyl-2-oxobuttersäure,
2-Oxovaleriansäüre,
Ketomalonsäure,
3-Methyl-2-oxovaleriansäure,
Trimethylbrenztraubensäure,
Oxolessigsäure,
2-Ketoglutarsäure,
Benzylameisensäure,
2-Oxooctansäure,
2-Oxoadipinsäure,
Phenylbrenztraübensäure,
Brombrenztraubensäure,
2-Ketopimelinsäure,
4-Hydroxyphenylbrenztraubensäure,
3-Indolglyoxalsäure,
Imidazölbrenztraubensäure HCl,
2-Keto-L-gulonsäure,
2-Carboxy-α-oxobenzolessigsäure,
Indol-3-brenztraubensäure,
2-Ketoglutarsäuredihydrat,
Brenztraubensäureamid,
N-Methylbrenztraubensäureamid,
N-Ethylbrenztraubensäureamid,
N,N-Dimethylbrenztraubensäureamid,
N-2-Hydroxyethylbrenztraubensäureamid
und deren Stereoisomeren und organischen oder anorganischen Salzen und Solvaten ausgewählt sind.

9. Verfahren nach einem der vorhergehenden Ansprüche, **dadurch gekennzeichnet, dass** die Verbindungen der Formel (V) aus :
Brenztraubensäure,
2-Ketobuttersäure,
β-Hydroxybrenztraubensäure,
Ketomalonsäure,
Oxolessigsäure,
2-Ketoglutarsäure,
2-Keto-L-gulonsäure,
2-Ketoglutarsäuredihydrat,
Brenztraubensäureamid
und deren Stereoisomeren und organischen oder anorganischen Salzen und Solvaten ausgewählt sind.

10. Verfahren nach einem der vorhergehenden Ansprüche, **dadurch gekennzeichnet, dass** es sich bei den Keratinfasern um Haare handelt.

11. Kit, umfassend mindestens:
- ein Erhitzungsmittel, das eine Temperatur zwischen 110 und 250°C liefert,
- eine Entkräuselungszusammensetzung, die mindestens zwei Entkräuselungsmittel gemäß einem der Ansprüche 1 und 7 bis 9 enthält.

## Claims

1. Process for relaxing keratin fibres, comprising:
(i) a step of applying to the keratin fibres a hair-relaxing composition having a pH of less than 9 and containing at least two denaturing agents,
- at least one of the denaturing agents is a guanidine corresponding to the general formula (III) below: 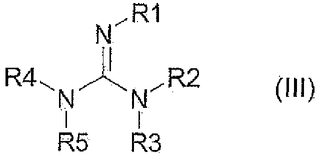 in which:
R1, R2, R3, R4 and R5 represent, independently:
(i) a hydrogen atom or
(ii) a linear or branched C1-C4 lower alkyl or alkenyl radical, optionally substituted with one or two radicals chosen from: hydroxyl, amino, dimethylamino, methoxy, ethoxy, carboxyl, carboxamide, N-methyl-carboxamide or SO₃H,
and the organic or mineral salts thereof,
- and at least one denaturing agent chosen from the α-keto acid derivatives corresponding to the general formula (V): 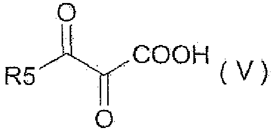
- R5 represents COOH, a linear or branched C1-C6 alkyl optionally substituted with an OH, COOH or Br radical; a phenyl or benzyl optionally substituted with an OH or COOH radical; or alternatively an indolyl radical or 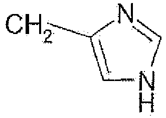
- and the stereoisomers, organic or mineral salts and solvates thereof,
(ii) a step of raising the temperature of the keratin fibres, using a heating means, to a temperature of between 110 and 250°C.

2. Process according to Claim 1, **characterized in that** the pH of the composition is less than 7.

3. Process according to Claim 1 or 2, **characterized in that** the hair-relaxing composition comprises an overall concentration of between 1M and 8 M and preferable between 2 M and 8 M of the said denaturing agents.

4. Process according to any one of Claims 1 to 3, **characterized in that** the temperature is raised using a heating means to a temperature of between 120°C and 220°C and more advantageously between 140°C and 220°C.

5. Process according to any one of the preceding claims, **characterized in that** the molar mass of the denaturing agents is between 40 and 600 g/mol.

6. Process according to any one of the preceding claims, **characterized in that** the composition is applied to wet keratin fibres.

7. Process according to any one of the preceding claims, **characterized in that** the compounds of formula (III) are chosen from:
- guanidine hydrochloride
- guanidine acetate
- guanidine sulfate
- guanidine carbonate
- guanidine bicarbonate
- guanidine phosphate
- guanidine sulfamate
- 1-methylguanidine hydrochloride
- 1,1-dimethylguanidine hydrochloride
- 1-ethylguanidine hydrochloride
- 1,1,3,3-tetramethylguanidine hydrochloride
- 2-tert-butyl-1,1,3,3-tetramethylguanidine hydrochloride.

8. Process according to any one of the preceding claims, **characterized in that** the compounds of formula (V) are chosen from:
pyruvic acid
2-ketobutyric acid
β-hydroxypyruvic acid
3-methyl-2-oxobutyric acid
2-oxovaleric acid
ketomalonic acid
3-methyl-2-oxovaleric acid
trimethylpyruvic acid
oxolacetic acid
2-ketoglutaric acid
benzylformic acid
2-oxooctanoic acid
2-oxoadipic acid
phenylpyruvic acid
bromopyruvic acid
2-ketopimelic acid
4-hydroxyphenylpyruvic acid
3-indoleglyoxalic acid
imidazolopyruvic acid HCl
2-keto-L-gulonic acid
2-carboxy-a-oxobenzeneacetic acid
3-indolepyruvic acid
2-ketoglutaric acid dihydrate
pyruvamide
N-methylpyruvamide
N-ethylpyruvamide
N,N-dimethylpyruvamide
N-2-hydroxyethylpyruvamide
and the stereoisomers, organic or mineral salts and solvates thereof.

9. Process according to any one of the preceding claims, **characterized in that** the compounds of formula (V) are chosen from:
pyruvic acid
2-ketobutyric acid
β-hydroxypyruvic acid
ketomalonic acid
oxolacetic acid
2-ketoglutaric acid
2-keto-L-gulonic acid
2-ketoglutaric acid dihydrate
pyruvamide
and the stereoisomers, organic or mineral salts and solvates thereof.

10. Process according to any one of the preceding claims, **characterized in that** the keratin fibres are hair.

11. Kit comprising at least:
- one heating means that affords a temperature of between 110 and 250°C,
- one hair-relaxing composition containing at least two denaturing agents as defined according to any one of Claims 1 and 7 to 9.
